## Europäisches Patentamt
## European Patent Office
## Office européen des brevets

(19)

(11) Veröffentlichungsnummer: **0 088 257**
**B1**

(12)

# EUROPÄISCHE PATENTSCHRIFT

(45) Veröffentlichungstag der Patentschrift:
16.01.85

(51) Int. Cl.⁴: **A 61 B 5/14**, A 61 B 17/32

(21) Anmeldenummer: 83101495.6

(22) Anmeldetag: 17.02.83

(54) **Blutlanzette.**

(30) Priorität: 09.03.82 DE 3208391

(43) Veröffentlichungstag der Anmeldung:
14.09.83 Patentblatt 83/37

(45) Bekanntmachung des Hinweises auf die Patenterteilung:
16.01.85 Patentblatt 85/3

(84) Benannte Vertragsstaaten:
AT BE CH DE FR GB IT LI LU NL SE

(56) Entgegenhaltungen:
EP - A - 0 017 999
EP - A - 0 061 102
DE - A - 2 130 438
DE - A - 2 909 349
FR - A - 2 150 994
GB - A - 975 050
US - A - 3 358 689

(73) Patentinhaber: Beiter, Werner, Daimlerstrasse 8,
D-7735 Dauchingen (DE)

(72) Erfinder: Beiter, Werner, Daimlerstrasse 8,
D-7735 Dauchingen (DE)

(74) Vertreter: Patentanwälte, Dipl.-Ing. Klaus Westphal Dr.
rer. nat. Bernd Mussgnug Dr. rer. nat. Otto Buchner,
Waldstrasse 33, D-7730 VS-Villingen (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Blutlanzette mit einem Griff und einer Spitze, die einstückig aus Kunststoff bestehen.

Blutlanzetten dienen dazu, durch einen kurzen Einstich, vorzugsweise in die Fingerkuppe, eine geringe Blutmenge, z. B. für Diagnosezwecke, zu entnehmen. In der Regel werden Einmal-Lanzetten verwendet, um die Infektionsgefahr zu vermeiden. Es sind Blutlanzetten gebräuchlich, die aus einem dünnen Metallplättchen ausgestanzt sind. Da eine Feinbearbeitung der Spitze aus Kostengründen nicht möglich ist, ist der Einstich mit einer solchen Blutlanzette verhältnismässig schmerzhaft und führt zu einem gewissen Nachbluten der Einstichwunde.

Es sind aus EP Nr. 001 7999 auch Blutlanzetten der eingangs genannten Gattung bekannt, die aus einem einstückig aus Kunststoff gespritzten Griff mit angeformter Spitze bestehen. Die Spitze dieser Blutlanzetten ist verhältnismässig stumpf, weil einerseits eine feine Spitze auf spritztechnische Schwierigkeiten stösst und andererseits eine feine Spitze während des Transports und der Lagerung leicht abbricht. Aufgrund der stumpfen Spitze erzeugen auch diese bekannten Kunststoff-Blutlanzetten eine schmerzhafte und nachblutende Einstichwunde.

Schliesslich ist aus US Nr. 3358689 eine Blutlanzette bekannt, die aus einer feinen Stahlnadel besteht, welche zur Halterung mit Kunststoff umspritzt ist. Zum Schutz der feinen Spitze ist diese von einer Kunststoff-Schutzkappe umschlossen, die einstückig mit der Halterung gespritzt ist und vor Gebrauch der Lanzette abgedreht wird. Die Herstellung der feinen Stahlnadel und deren Umspritzen mit Kunststoff macht diese Blutlanzette sehr aufwendig.

Der Erfindung liegt die Aufgabe zugrunde, eine preisgünstige Blutlanzette zu schaffen, die einen möglichst schmerzfreien und nicht nachblutenden Einstich ermöglicht.

Diese Aufgabe wird bei einer Blutlanzette der eingangs genannten Gattung erfindungsgemäss dadurch gelöst, dass ein flacher Schutzbügel vorgesehen ist, der beiderseits der Spitze einstückig an dem Griff angeformt ist, und dass die Spitze in einer den Schutzbügel senkrecht zu seiner Flächenausdehnung durchsetzenden Ausnehmung aufgenommen ist.

Vorzugsweise sind die beiden Ansatzstellen des Schutzbügels an dem Griff als Sollbruchstellen ausgebildet.

Vorzugsweise weist die Spitze einen in einer ihrer Querachsen langgestreckten Querschnitt auf, der sich in der Richtung der zu dieser senkrechten anderen Querachse gegen die Mitte des Querschnitts verbreitert. Insbesondere ist dabei die Spitze im Querschnitt rhombisch und weist scharfe Seitenkanten auf.

Zweckmässig kann die Spitze einen Sockel mit grossem Pyramidenöffnungswinkel und eine abgesetzte Einstechspitze mit kleinem Pyramidenöffnungswinkel aufweisen. Dabei können der Griff und der Schutzbügel im wesentlichen den gleichen flachen Querschnitt aufweisen und ihre Dicke der kleineren Diagonalen der rhombischen Grundfläche der Spitze entsprechen.

Vorzugsweise ist der Abstand zwischen der Spitze und dem Schutzbügel kleiner als die Dicke des Schutzbügels.

Vorzugsweise setzt sich die Ausnehmung axial an die Spitze anschliessend in einen Schlitz fort, der in eine den Schutzbügel durchsetzende Entlüftungsbohrung mündet.

Der Griff und der Schutzbügel können Griffmulden aufweisen.

Zweckmässig weist der Griff einen dünnen mittleren Bereich und einen verstärkten Umfangsrand auf, wobei die Griffmulden ggf. in diesem Umfangsrand ausgebildet sind.

Die erfindungsgemässe Blutlanzette besteht vollständig aus Kunststoff und ist in einem Stück im Spritzgussverfahren hergestellt. Durch die flache Ausgestaltung der gesamten Blutlanzette ergibt sich zudem auch ein geringer Materialbedarf, so dass die Blutlanzette insgesamt sehr preisgünstig hergestellt werden kann.

Der die Spitze umschliessende Schutzbügel verhindert eine Beschädigung der Spitze während des Transports und der Lagerung, so dass die Spitze sehr fein ausgebildet sein kann, wodurch sich ein feiner Einstich ergibt, der wenig schmerzhaft ist und keine nachblutende Wunde zur Folge hat.

In vorteilhafter Weise ist die Spitze abgesetzt ausgebildet. Sie weist einen breiten Sockel auf, der mit grossem Pyramidenöffnungswinkel, d.h. stumpf zuläuft, und eine feine eigentliche Einstechspitze, die mit kleinem Pyramidenöffnungswinkel, d. h. spitz zuläuft. Der eigentliche Einstich erfolgt dabei nur durch die feine Einstechspitze, während der breitere Sockel dieser Spitze die nötige mechanische Festigkeit verleiht und die Einstechtiefe begrenzt. Dadurch wird ein feiner, wenig schmerzhafter und nicht nachblutender Einstich möglich.

Spritztechnisch wird die Ausbildung der feinen Spitze insbesondere dadurch begünstigt, dass die die Spitze aufnehmende Ausnehmung des Schutzbügels im Bereich der Spitze über einen Schlitz mit einer Entlüftungsbohrung in Verbindung steht.

Die Spritzgussform kann daher unmittelbar an der feinen Spitze entlüftet werden, so dass das Kunststoffmaterial sauber und vollständig in die feine Spitze fliesst.

Der Schutzbügel ist flach ausgebildet und umgibt die Spitze nur längs deren Umfangslinie. Der Schutzbügel kann daher sehr bequem durch Abbiegen senkrecht zu seiner Flächenausdehnung abgebrochen werden, um die Blutlanzette gebrauchsbereit zu machen. Das Abbrechen des Schutzbügels wird noch dadurch erleichtert, dass seine Ansatzstellen an dem Griff als Sollbruchstellen mit geringer Materialstärke ausgebildet sind.

Obwohl der Schutzbügel die Spitze nur längs deren Umfang umgibt, ist ein vollständiger und zuverlässiger Schutz der Spitze gegen Beschädigung dadurch gewährleistet, dass der Abstand zwischen der Umfangskante der Spitze und dem Schutzbügel kleiner als die Dicke des Schutzbügels gewählt ist. Der Schutzbügel deckt auf diese Weise auch senkrecht zu seiner Flächenausdehnung die besonders empfindlichen Bereiche der Spitze, nämlich die vordere punktförmige Einstechspitze und die den Einschnitt bewirkenden, scharfen Seitenkanten der im Querschnitt rhombischen Spitze, ausreichend ab.

Im folgenden wird die Erfindung anhand eines in der Zeichnung dargestellten Ausführungsbeispiels näher erläutert. Es zeigen:

Fig. 1    eine Draufsicht auf eine Blutlanzette,

Fig. 2    einen Schnitt gemäss der Linie A-B in Fig. 1,

Fig. 3    einen Schnitt gemäss der Linie C-D in Fig. 1,

Fig. 4    einen Schnitt gemäss der Linie E-F in Fig. 1, und

Fig. 5    einen Schnitt gemäss der Linie G-H in Fig. 1.

Die in der Zeichnung stark vergrössert dargestellte Blutlanzette ist in einem Stück im Spritzgussverfahren aus Kunststoff hergestellt.

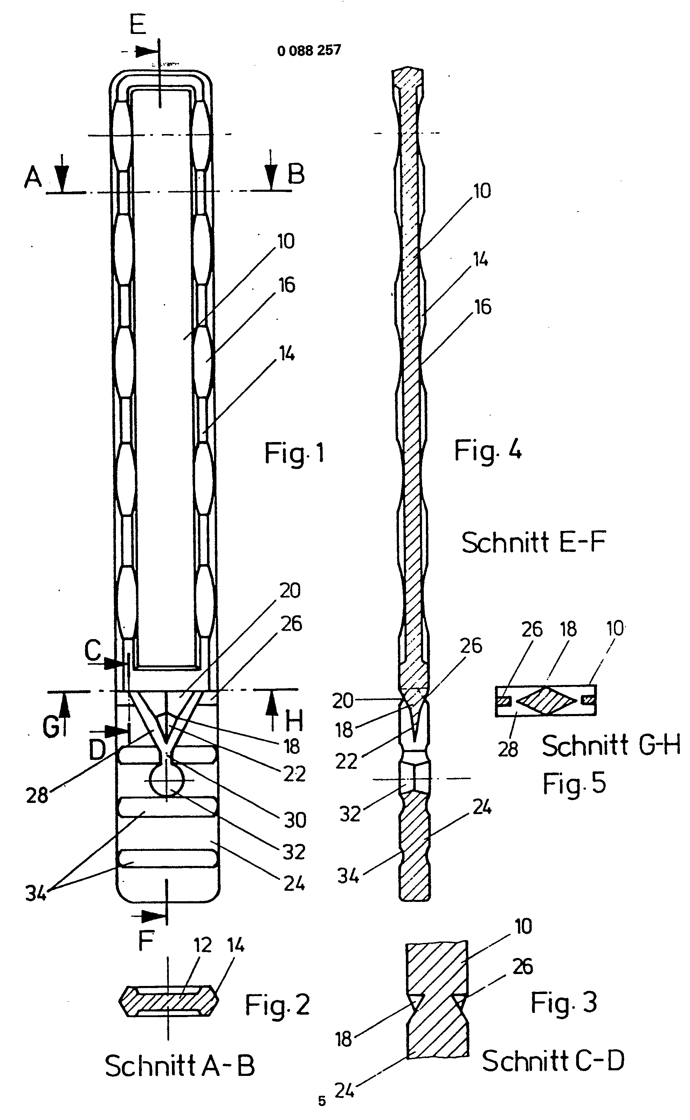

Die Blutlanzette weist einen Griff 10 mit einem flachen Querschnitt, wie ihn Fig. 2 zeigt, auf. Im mittleren Bereich 12 weist der Griff 10 eine geringe Dicke auf, während sein Umfangsrand 14 verstärkt ist. Dadurch ergibt sich ein geringer Materialbedarf bei hoher mechanischer Festigkeit und Steifigkeit. In dem verstärkten Umfangsrand 14 sind Griffmulden 16 ausgebildet, die ein zuverlässiges Erfassen und Festhalten der Blutlanzette gewährleisten.

An der vorderen Stirnfläche des Griffs 10 ist in einem Stück eine Spitze 18 angeformt. Wie Fig. 5 zeigt, weist die Spitze 18 im Querschnitt die Form eines flachen, langgestreckten Rhombus auf. Die Spitze 18 setzt an dem Griff 10 mit einem breiten Sockel 20 an, der mit einem verhältnismässig grossen Kegelöffnungswinkel stumpf zuläuft. An den Sockel 20 schliesst sich abgesetzt die eigentliche Einstechspitze 22 an, die mit kleinerem Kegelöffnungswinkel spitz zuläuft.

Wie Fig. 5 zeigt, entspricht die kürzere Diagonale der Grundfläche der Spitze 18 der Dicke des Umfangsrandes 14 des Griffes 10, so dass eine optimale mechanische Stabilität der Spitze erreicht wird. In der Breitenausdehnung des Griffes 10 ist der rhombische Querschnitt der Spitze 18 langgestreckt, so dass sich spitzwinklige Schneidkanten der Spitze 18 ergeben. An der vorderen Stirnfläche des Griffes 10 ist weiter einstückig ein Schutzbügel 24 angeformt. Der Schutzbügel 24 entspricht im Querschnitt im wesentlichen dem Griff 10. Der Schutzbügel 24 setzt beiderseits der Spitze 18 am Rand des Griffes 10 an. An den Ansatzstellen ist die Materialstärke des Schutzbügels verringert, so dass sich Sollbruchstellen 26 ergeben.

Im Bereich der Spitze 18 weist der Schutzbügel 24 eine senkrecht zu seiner Flächenausdehnung durchgehende Ausnehmung 28 auf, die, wie Fig. 1 zeigt, in ihrer Form der Projektion der Spitze 18 in die Ebene des Griffes 10 entspricht. Die Abmessungen der Ausnehmung 18 sind so gewählt, dass der Abstand der Schneidkanten der Spitze 18 von dem Schutzbügel 24, d. h. von dem Rand der Ausnehmung 28, kleiner ist als die Materialdicke des Schutzbügels 24.

An ihrem vorderen spitzen Ende läuft die Ausnehmung 28 in einen axialen Schlitz 30 aus, der seinerseits in eine den Schutzbügel 24 durchsetzende Entlüftungsbohrung 32 mündet. Über den Schlitz 30 und die Entlüftungsbohrung 32 kann die Spritzgussform beim Giessen der feinen Einstechspitze 22 entlüftet werden.

Der Schutzbügel 24 weist vor der Spitze 18 ebenfalls Griffmulden 34 auf. Um die Blutlanzette gebrauchsbereit zu machen, wird der Schutzbügel im Bereich vor der Spitze 18 erfasst, was durch die Griffmulden 34 begünstigt wird und durch Abbiegen aus der Zeichenebene der Fig. 1 heraus längs der Sollbruchstellen 26 abgebrochen.

## Patentansprüche

1.    Blutlanzette mit einem Griff und einer Spitze, die einstückig aus Kunststoff bestehen, dadurch gekennzeichnet, dass ein flacher Schutzbügel (24) vorgesehen ist, der beiderseits der Spitze (18) einstückig an dem Griff (10) angeformt ist, und dass die Spitze (18) in einer den Schutzbügel (24) senkrecht zu seiner Flächenausdehnung durchsetzenden Ausnehmung (28) aufgenommen ist.

2.    Blutlanzette nach Anspruch 1, dadurch gekennzeichnet, dass die beiden Ansatzstellen des Schutzbügels (24) an dem Griff (10) als Sollbruchstellen (26) ausgebildet sind.

3.    Blutlanzette nach einem der beiden Ansprüche 1 oder 2, dadurch gekennzeichnet, dass die Spitze (18) einen in einer ihrer Querachsen langgestreckten Querschnitt aufweist, der sich in der Richtung der zu dieser senkrechten anderen Querachse gegen die Mitte des Querschnitts verbreitert.

4.    Blutlanzette nach Anspruch 3, dadurch gekennzeichnet, dass die Spitze (18) im Querschnitt rhombisch ist und scharfe Seitenkanten aufweist.

5.    Blutlanzette nach Anspruch 3, dadurch gekennzeichnet, dass die Spitze (18) einen Sockel (20) mit grossem Pyramidenöffnungswinkel und eine abgesetzte Einstechspitze (22) mit kleinem Pyramidenöffnungswinkel aufweist.

6.    Blutlanzette nach einem der beiden Ansprüche 4 oder 5, dadurch gekennzeichnet, dass der Griff (10) und der Schutzbügel (24) im wesentlichen den gleichen flachen Querschnitt aufweisen und ihre Dicke der kleineren Diagonalen der rhombischen Grundfläche der Spitze (18) entspricht.

7.    Blutlanzette nach einem der vorhergehenden Ansprüche, dadurch gekennzeichnet, dass der Abstand zwischen der Spitze (18) und dem Schutzbügel (24) kleiner ist als die Dicke des Schutzbügels.

8.    Blutlanzette nach einem der vorhergehenden Ansprüche, dadurch gekennzeichnet, dass die Ausnehmung (28) sich axial an die Spitze (18) anschliessend in einen Schlitz (30) fortsetzt, der in eine den Schutzbügel (24) durchsetzende Entlüftungsbohrung (32) mündet.

9. Blutlanzette nach einem der vorhergehenden Ansprüche, dadurch gekennzeichnet, dass der Griff (10) und der Schutzbügel (24) Griffmulden (16 bzw. 34) aufweisen.

10. Blutlanzette nach einem der vorhergehenden Ansprüche, dadurch gekennzeichnet, dass der Griff (10) einen dünnen mittleren Bereich (12) und einen verstärkten Umfangsrand (14) aufweist, wobei die Griffmulden (16) ggf. in diesem Umfangsrand (14) ausgebildet sind.

## Claims

1. A surgical lancet having a handle and a point and which is made in one piece of a plastics material, characterised in that a flat protective guard (24) on both sides of the point (18) is moulded integrally with the handle (10), and the point (18) is located in an aperture which extends through the protective guard (24) at right angles to its flat extension.

2. A lancet according to Claim 1, characterised in that both junctions of the protective guard (24) with the handle (10) are formed as predetermined breaking points (26).

3. A lancet according to Claim 1 or 2, characterised in that the cross-section of the point (18) is elongated in the direction of one of its transverse axes, and widens towards the middle of the cross-section in the direction of the other transverse axis perpendicular to the first mentioned axis.

4. A lancet according to Claim 3, characterised in that the point (18) has a rhombic cross-section and sharp side edges.

5. A lancet according to Claim 3, characterised in that the point (18) has a base (20) with a large pyramidal angle and an offset percing point (22) with a small pyramidal angle.

6. A lancet according to Claim 4 or 5, characterised in that the handle (10) and the protective guard (24) have substantially the same flat cross-section and their thickness corresponds to the smaller diagonal of the rhombic base area of the point (18).

7. A lancet according to any one of the preceding claims, characterised in that the distance between the point (18) and the protective guard (24) is smaller than the thickness of the protective guard.

8. A lancet according to any one of the preceding claims, characterised in that the aperture (28) is extended axially adjacent to the point (18) as a slot (30) which leads into a venting bore (32) which passes through the protective guard (24).

9. A lancet according to any one of the preceding claims, characterised in that the handle (10) and the protective guard (24) have gripping depressions (16 and 34 respectively).

10. A lancet according to any one of the preceding claims, characterised in that the handle (10) has thin middle region (12) and a strengthened peripheral rim (14), the gripping depressions (16), if appropriate, being formed in this peripheral rim (14).

## Revendications

1. Lancette pour prise de sang comportant un manche et une pointe constitués d'une seule pièce en matière plastique, caractérisée en ce qu'on prévoit un étrier de protection plat (24) qui est moulé d'une seule pièce sur le manche (10) de chaque côté de la pointe (18), et que la pointe (18) est logée dans un évidement (28) traversant l'étrier de protection (24) perpendiculairement à sa surface.

2. Lancette selon la revendication 1, caractérisée en ce que les deux points d'attache de l'étrier de protection (24) sont réalisés sur le manche (10) sous forme de zones de rupture (26).

3. Lancette selon l'une des revendications 1 ou 2, caractérisée en ce que la pointe (18) a une section transversale allongée dans le sens de l'un de ses axes transversaux et que cette section s'élargit vers son milieu dans le sens de l'autre axe transversal perpendiculaire au précédent.

4. Lancette selon la revendication 3, caractérisée en ce que la pointe (18) a une section transversale rhomboïdale et comporte des arêtes de coupe tranchantes.

5. Lancette selon la revendication 3, caractérisée en ce que la pointe (18) comporte une base (20) ayant un grand angle d'ouverture de la pyramide et une pointe d'incision (22) en décrochement ayant un petit angle d'ouverture de la pyramide.

6. Lancette selon l'une des revendications 4 ou 5, caractérisée en ce que la poignée (10) et l'étrier de protection (24) ont sensiblement la même section transversale plate et que leur épaisseur correspond à la petite diagonale de la surface de base rhomboïdale de la pointe (18).

7. Lancette selon l'une des revendications précédentes, caractérisée en ce que la distance entre la pointe (18) et l'étrier de protection (24) est inférieure à l'épaisseur de l'étrier de protection.

8. Lancette selon l'une des revendications précédentes, caractérisée en ce que l'évidement (28) se prolonge en se raccordant axialement à la pointe (18) par une fente (30) qui débouche dans un évent de mise à air libre (32) traversant l'étrier de protection (24).

9. Lancette selon l'une des revendications précédentes, caractérisée en ce que le manche (10) et l'étrier de protection (24) comportent des creux de préhension (16, 34).

10. Lancette selon l'une des revendications précédentes, caractérisée en ce que le manche (10) comporte une zone médiane mince (12) et un bord périphérique renforcé (14), les creux de préhension étant éventuellement formés dans ce bord périphérique (14).

0 088 257

Fig. 1

Fig. 4

Schnitt E-F

Schnitt G-H
Fig. 5

Fig. 2

Schnitt A-B

Fig. 3

Schnitt C-D